Europäisches Patentamt

European Patent Office

Office européen des brevets

(11) Veröffentlichungsnummer : **0 014 305**
**B1**

# (12) EUROPÄISCHE PATENTSCHRIFT

(45) Veröffentlichungstag der Patentschrift :
13.10.82

(21) Anmeldenummer : 80100022.5

(22) Anmeldetag : 04.01.80

(51) Int. Cl.³ : **C 07 C 19/045, C 07 C 17/38//**
**C07C21/06, C07C17/34**

(54) **Verfahren zur Reinigung von 1,2-Dichloräthan, das bei der unvollständigen thermischen Spaltung zu Vinylchlorid zurückgewonnen wurde.**

(30) Priorität : 31.01.79 DE 2903640

(43) Veröffentlichungstag der Anmeldung :
20.08.80 (Patentblatt 80/17)

(45) Bekanntmachung des Hinweises auf die Patenterteilung : 13.10.82 Patentblatt 82/41

(84) Benannte Vertragsstaaten :
BE DE FR GB IT NL

(56) Entgegenhaltungen :
DE A 1 917 933
DE A 1 959 211
DE A 2 307 376
DE A 2 747 523
DE B 2 416 786

(73) Patentinhaber : HOECHST AKTIENGESELLSCHAFT
Postfach 80 03 20
D-6230 Frankfurt/Main 80 (DE)

(72) Erfinder : Rechmeier, Gerhard, Dr.
Berthold-Brecht-Strasse 66
D-5042 Erftstadt (DE)
Erfinder : Roesnik, Ulrich
Bergstrasse 73
D-5030 Hürth (DE)
Erfinder : Scholz, Harald, Dr.
Am Beissel 8
D-5042 Erftstadt (DE)

# Verfahren zur Reinigung von 1,2-Dichloräthan, das bei der unvollständigen thermischen Spaltung zu Vinylchlorid zurückgewonnen wurde

Die Herstellung von Vinylchlorid durch unvollständige thermische Spaltung von 1,2-Dichloräthan ist z.B. aus den DE-PS 857 957 und 899 191 sowie der US-PS 2 724 006 und der GB-PS 938 824 bekannt. Der bei der thermischen Spaltung nicht umgesetzte 1,2-Dichloräthan-Anteil, in der Regel 40 bis 60 %, muß vor seiner Wiederverwendung in der Spaltanlage von Verunreinigungen befreit werden. Diese Reinigung muß sehr sorgfältig vorgenommen werden, um die Laufzeit der beheizten Schlange im Pyrolysereaktor so lang wie möglich auszudehnen. Die Verunreinigungen in dem zur Spaltung zurückgeführten 1-2-Dichloräthan werden auch in geringer Konzentration bevorzugt zu kohlenstoff abgebaut, der sich sowohl in der beheizten Schlange im Pyrolysereaktor als auch in den nachgeschalteten Apparaturen ablagert. Dies führt zu Reinigungsarbeiten, die mit Kosten, Produktverlust und Kapazitätsminderung verbunden sind.

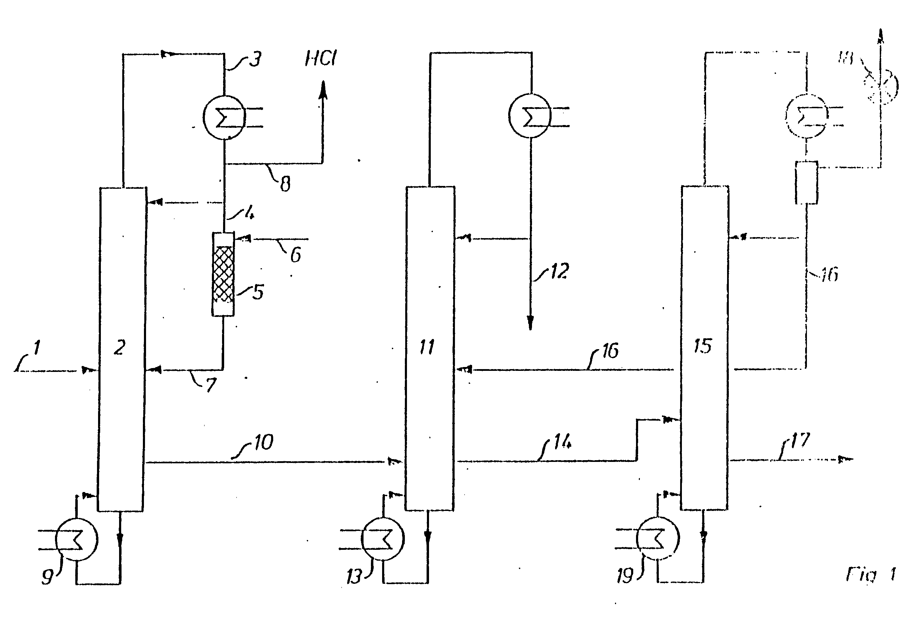

In der US-PS 2 748 176 und der DE-PS 1 917 933 wird vorgeschlagen, die im zurückgewonnenen 1,2-Dichloräthan enthaltenen leichtsiedenden Verunreinigungen zu Hochsiedern zu chlorieren. Hiervon macht beispielsweise folgende betriebliche Arbeitsweise Gebrauch (vgl. Figur 1):

Das aus der Pyrolyse zurückgewonnene 1,2-Dichloräthan, das frei von Chlorwasserstoff und Vinylchlorid ist, aber noch unterhalb 83,7 °C (1 011 mbar) siedende Leichtsieder und oberhalb 83,7 °C (1 011 mbar) siedende Hochsieder enthält, gelangt über Leitung (1) in den mittleren Teil der unter Normaldruck betriebenen Leichtsiederkolonne (2), wobei die Leichtsieder über die Kopfleitung (3) abdestillieren, kondensieren und teilweise wieder auf den Kopf der Kolonne (2) zurückgeführt werden. Ein Teilstrom (4) des Kondensats gelangt jedoch in einen mit Füllkörpern aus Eisen beschickten Behälter (5), dem über die Leitung (6) gasförmiges Chlor zugeführt wird. Im Behälter (5) werden die Leichtsieder bei 30-85 °C in Gegenwart von aus Eisen und Chlor entstehendem Eisenchlorid als Katalysator zu Hochsiedern chloriert. Der Ablauf (7) des Chlorierungsbehälters (5) wird etwa in die Mitte der Kolonne (2) zurückgeführt. Bei der Chlorierung entstehender Chlorwasserstoff kann schließlich unterhalb des Kondensators über Leitung (8) entweichen. Die Kolonne (2) wird durch einen Umlaufverdampfer (9) beheizt. Der Sumpf der Leichtsiederkolonne (2) gelangt über die Leitung (10) in die unter Normaldruck betriebene Hochsiederkolonne (11), worin reines 1,2-Dichloräthan über Kopf abdestilliert, kondensiert und über Leitung (12) abgezogen wird. Die Kolonne (11) wird durch einen Umlaufverdampfer (13) beheizt. Der Sumpf der Hochsieder-Kolonne (11) gelangt über die Leitung (14) in die Vakuumkolonne (15), worin über Kopf restliches 1,2-Dichloräthan abdestilliert, kondensiert und über Leitung (16) in die Hochsiederkolonne (11) zurückgeführt wird.

Der aus Hochsiedern bestehende Sumpf der Kolonne (15) wird über Leitung (17) diskontinuierlich abgezogen und verbrannt. Der in der Kolonne (15) herrschende Unterdruck wird durch die Vakuumpumpe (18) erzeugt. Die Kolonne (15) wird durch einen Umlaufverdampfer (19) beheizt. Beispiel 1 erläutert diesen betrieblichen Stand der Technik.

Dieses Verfahren hat den Nachteil, daß die bei der Chlorierung im Behälter (5) entstandenen festen Nebenbestandteile, hauptsächlich Eisenchlorid und ein wenig Kohlenstoff, die Kolonne (2) verstopfen. Die Laufzeit der Kolonne (2) und ihres Umlaufverdampfers (9) ist entsprechend kurz. Durch das unregelmäßige Arbeiten bei beginnender Verstopfung können leichtsiedende Verunreinigungen über Leitung (10) in die Hochsiederkolonne (11) und von dort über die Kopfleitung (12) in die Pyrolyse gelangen.

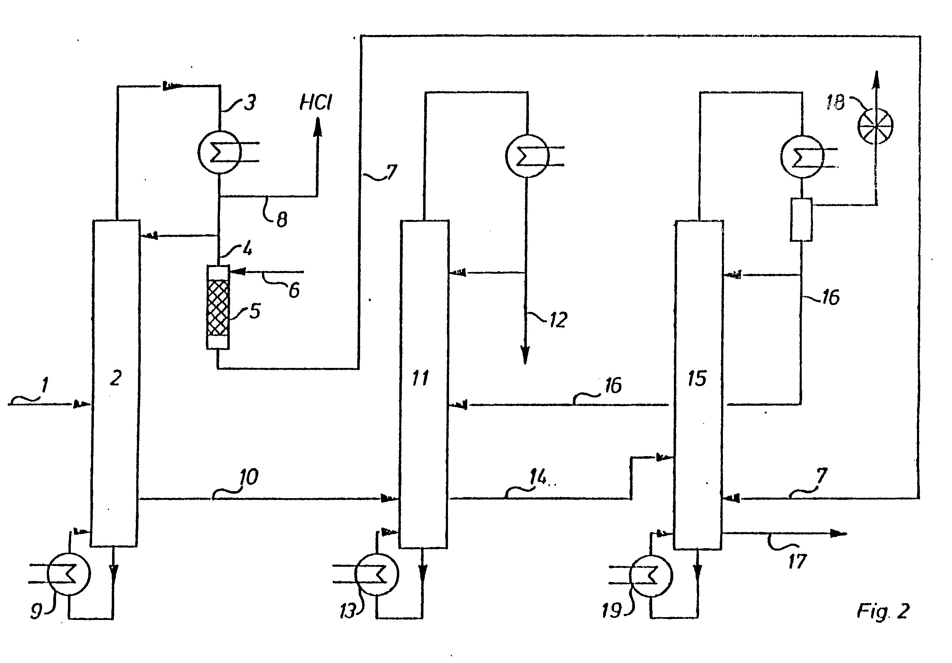

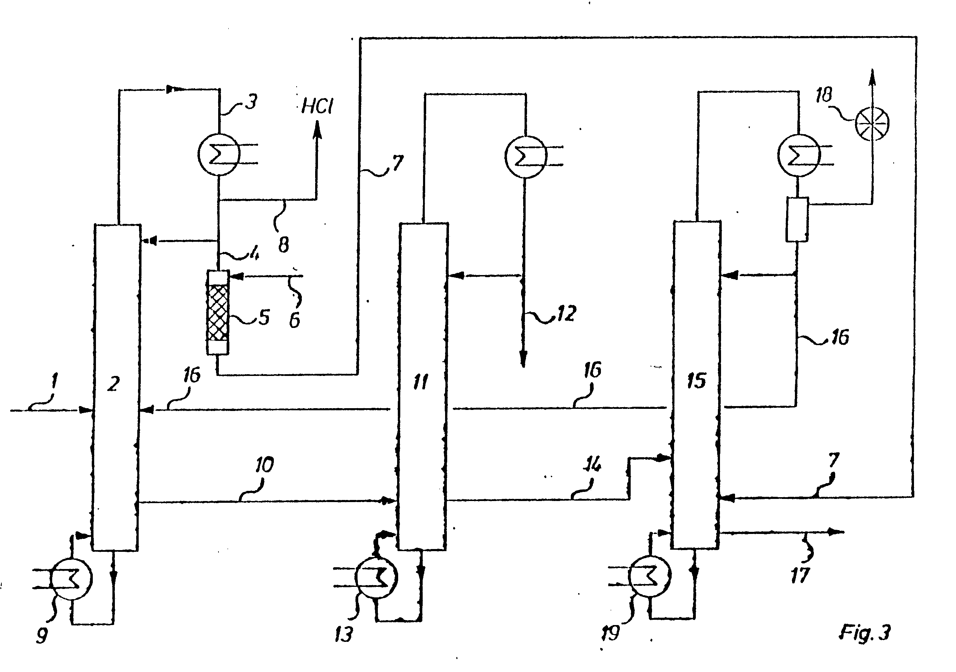

Es wurde nun überraschenderweise gefunden, daß die Verstopfung der Kolonne (2) verhindert werden kann, wenn man den Ablauf (7) des Chlorierungsbehälters (5) in den unteren Teil der Vakuumkolonne (15) münden läßt, wie dies z.B. Figur 2 zeigt, die abgesehen von dieser Änderung mit Figur 1 identisch ist. Die festen Verunreinigungen werden dann direkt über Leitung (17) aus der Vakuumkolonne (15) kontinuierlich ausgeschieden. Neben der Laufzeitverbesserung der Leichtsiederkolonne (2) ergibt sich auch eine Qualitätsverbesserung des 1,2-Dichloräthans im Ablauf (12) der Hochsiederkolonne (11). Beispiel 2 erläutert das Verfahren der Erfindung.

Der Ablauf (16) der Vakummkolonne (15), die bevorzugt bei Drucken von 200 bis 400 mbar und Blasentemperaturen von 75-95 °C arbeitet, wird jedoch durch Chlorwasserstoff als Folge einer geringfügigen Spaltung von Hochsiedern während der Vakuumdestillation verunreinigt. Auch entweicht ein Teil des Chlorwasserstoffs, der bei der Chlorierung der Leichtsieder im Behälter (5) entsteht, nicht über die Abgasleitung (8), sondern gelangt über die Leitung (7) in die Vakuumkolonne (15) (Figur 2). Gemäß den Figuren 1 und 2 gelangt das verunreinigte 1,2-Dichloräthan aus dem Ablauf (16) zurück in die Hochsiederkolonne (11), wo die unterhalb 83,7 °C (1 011 mbar) siedenden Leichtsieder über die Kopfleitung (12) in die Pyrolyse gelangen können.

Auch dieser Nachteil kann durch ein weiteres Merkmal der Erfindung vermieden werden, wenn man gemäß Figur 3, die abgesehen von dieser Änderung mit Figur 2 identisch ist, den Ablauf (16) etwa in die Mitte der Leichtsiederkolonne (2) zurückführt, da im Bereich der Leichtsiederkolonne die Chlorierung der Leichtsieder zu Hochsiedern stattfindet. Beispiel 3 erläutert diesen zusätzlichen Verfahrensschritt gemäß der Erfindung.

Im einzelnen betrifft die Erfindung nunmehr ein Verfahren zur Reinigung von 1,2-Dichloräthan,

das bei der unvollständigen thermischen Spaltung zu Vinylchlorid zurückgewonnen wurde und unterhalb 83,7 °C (1 011 mbar) siedende Leichtsieder sowie oberhalb 83,7 °C (1 011 mbar) siedende Hochsieder als Verunreinigungen enthält, wobei man in einer ersten Destillationszone über Kopf die Leichtsieder aus dem verunreinigten 1,2-Dichloräthan abdestilliert, laufend einen Teil des Leichtsiederkonzentrates durch Begasung mit Chlor bei 30 bis 85 °C in Hochsieder umwandelt, in einer zweiten Destillationszone reines 1,2-Dichloräthan über Kopf von den Hochsiedern abdestilliert und in einer dritten Destillationszone unter vermindertem Druck restliches 1,2-Dichloräthan aus dem Hochsiederkonzentrat abdestilliert und die Hochsieder ausschleust, welches dadurch gekennzeichnet ist, daß man den hochsiederhaltigen Ablauf aus der Chlorierung des Leichtsiederkonzentrats in den unteren Teil der dritten Destillationszone einführt.

Besonders vorteilhaft ist es dabei, das 1,2-Dichloräthan enthaltende Destillat der dritten Destillationszone in den mittleren Teil der ersten Destillationszone zurückzuführen. Diese Maßnahme ist selbst dann vorteilhaft, wenn man den hochsiederhaltigen Ablauf aus der Chlorierung des Leichtsiederkonzentrats in bekannter Weise etwa in die Mitte der ersten Destillationszone zurückführt.

Beispiel 1 (Vergleichsbeispiel gemäß Figur 1)

36 000 kg/h 1,2-Dichloräthan werden einer Pyrolyseanlage zugeführt, wobei eine Spaltrate von 50,5 % eingestellt wird. 17 820 kg/h 1,2-Dichloräthan werden zurückgewonnen und müssen destillativ gereinigt werden. Zu diesem Zweck wird aus dem Rückfluß der Leichtsiederkolonne (2) ein Teilstrom (4) von 400 l/h entnommen, mit 20 kg/h Chlor aus Leitung (6) im Chlorierungsbehälter (5) behandelt und über Leitung (7) in die Kolonne (2) zurückgeführt.

Die Laufzeit der Leichtsiederkolonne (2) betrug 65 Tage, die des zugehörigen Umlaufverdampfers (9) 215 Tage.

Das aus der Hochsiederkolonne (11) über die Kopfleitung (12) anfallende 1,2-Dichloräthan hatte eine Reinheit von 98,8 %.

Der Druckanstieg in der Pyrolyseschlange infolge Verkokung betrug 45,55 mbar/Tag.

Beispiel 2 (gemäß Figur 2)

Man arbeitet wie in Beispiel 1, führt jedoch den Ablauf (7) des Chlorierungsbehälters (5) in den unteren Teil der Vakuumkolonne (15) ein.

Die Laufzeit der Leichtsiederkolonne (2) erhöhte sich dadurch auf 730 Tage, die des zugehörigen Umlaufverdampfers (9) auf 580 Tage.

Das aus der Hochsiederkolonne (11) über die Kopfleitung (12) anfallende 1,2-Dichloräthan hatte eine Reinheit von 99,2 %.

Der Druckanstieg in der Pyrolyseschlange infolge Verkokung betrug nur noch 21,15 mbar/Tag.

Beispiel 3 (gemäß Figur 3)

Man arbeitet wie in Beispiel 2, führt jedoch den Ablauf (16) der Vakuumkolonne (15) in den mittleren Teil der Leichtsiederkolonne (2) ein.

Die Laufzeit der Leichtsiederkolonne (2) und ihres Umlaufverdampfers (9) betrugen unverändert 730 bzw. 580 Tage. Das aus der Hochsiederkolonne (11) über die Kopfleitung (12) anfallende 1,2-Dichloräthan hatte eine Reinheit von 99,6 %. Der Druckanstieg in der Pyrolyseschlange infolge Verkokung sank weiter auf unter 10,25 mbar/Tag.

**Ansprüche**

1. Verfahren zur Reinigung von 1,2-Dichloräthan, das bei der unvollständigen thermischen Spaltung zu Vinylchlorid zurückgewonnen wurde und unterhalb 83,7 °C (1 011 mbar) siedende Leichtsieder sowie oberhalb 83,7 °C (1 011 mbar) siedende Hochsieder als Verunreinigungen enthält, wobei man in einer ersten Destillationszone über Kopf die Leichtsieder aus dem verunreinigten 1,2-Dichloräthan abdestilliert, laufend einen Teil des Leichtsiederkonzentrates durch Begasung mit Chlor bei 30 bis 85 °C in Hochsieder umwandelt, in einer zweiten Destillationszone reines 1,2-Dichloräthan über Kopf von den Hochsiedern abdestilliert und in einer dritten Destillationszone unter vermindertem Druck restliches 1,2-Dichloräthan aus dem Hochsiederkonzentrat abdestilliert und die Hochsieder ausschleust, dadurch gekennzeichnet, daß man den hochsiederhaltigen Ablauf aus der Chlorierung des Leichtsiederkonzentrats in den unteren Teil der dritten Destillationszone einführt.

2. Verfahren nach Anspruch 1, dadurch gekennzeichnet, daß man das 1,2-Dichloräthan enthaltende Destillat der dritten Destillationszone in den mittleren Teil der ersten Destillationszone zurückführt.

**Claims**

1. Process for purifying 1,2-dichloroethane which is recovered during incomplete thermal cracking to vinyl chloride and contains as contaminants low boilers boiling at a temperature lower than 83.7 °C (1 011 mbar) and high boilers boiling at a temperature higher than 83.7 °C (1 011 mbar), wherein the low boilers are distilled off overhead from contaminated 1,2-dichloroethane, in a first distilling zone ; a portion of low boiler concentrate is continuously treated at 30 to 85 °C with gaseous chlorine and converted to high boilers ; pure 1,2-dichloroethane is distilled off overhead from the high boilers, in a second distilling zone ; and residual 1,2-dichloroethane is distilled off under reduced pressure from the high boiler concentrate, in a third distilling zone, and the high boilers are removed, characterized in that the high boiler-containing effluent coming from the chlorination of the low boiler concentrate is introduced into the lower portion of the third distilling zone.

2. Process as claimed in Claim 1, wherein the distillate containing 1,2-dichloroethane coming from the third distilling zone is recycled to the center portion of the first distilling zone.

**Revendications**

1. Procédé pour la purification de 1,2-dichloroéthane qui a été récupéré dans la dissociation thermique incomplète en chlorure de vinyle et qui contient comme impuretés des produits volatils bouillant au-dessous de 83,7 °C (1 011 mbar), ainsi que des produits de haut point d'ébullition bouillant au-dessus de 83,7 °C (1 011 mbar), dans lequel on sépare en tête d'une première zone de distillation les produits volatils du 1,2-dichloroéthane contaminé, on transforme en continu une partie du concentrat de produits volatils en produits de haut point d'ébullition par traitement par le chlore gazeux à 30-85 °C, on sépare par distillation le 1,2-dichloroéthane pur des produits de haut point d'ébullition en tête dans une seconde zone de distillation et on sépare par distillation sous pression réduite le reste du 1,2-dichloroéthane du concentrat de produits de haut point d'ébullition dans une troisième zone de distillation et on écluse au-dehors les produits de haut point d'ébullition, caractérisé en ce que l'on introduit l'écoulement contenant les produits de haut point d'ébullition de la chloruration du concentrat des produits volatils dans la partie inférieure de la troisième zone de distillation.

2. Procédé selon la revendication 1, caractérisé en ce que l'on renvoie dans la partie moyenne de la première zone de distillation le distillat contenant le 1,2-dichloroéthane de la troisième zone de distillation.

Fig 1

HCl

Fig. 2

HCl

Fig. 3